# EUROPÄISCHE PATENTANMELDUNG

(11) **EP 0 582 800 A1**
(43) Veröffentlichungstag der Anmeldung: **16.02.1994**
(21) Anmeldenummer: 93108987.4
(22) Anmeldetag: 04.06.1993
(51) Int. Cl.: G02C 7/10, A61F 9/02

(54) **Optisches Glas für Sport-Brille, insbesondere Ski-Brillen, Verfahren für seine Herstellung sowie Sport-Brille mit wenigstens einem derartigen optischen Glas**

(30) Priorität: 13.06.1992 DE 4219389; 03.06.1993 DE 4318483
(71) Anmelder: Münch, Werner, W-93055 Regensburg (DE)
(72) Erfinder: Münch, Werner, W-93055 Regensburg (DE)
(74) Vertreter: Graf, Helmut, Dipl.-Ing.

(57) **Zusammenfassung**

Die Erfindung betrifft ein neuartiges optisches Glas für eine Skibrille bestehend aus mehreren Schichten oder nichtleitenden Elementen, sowie ein Verfahren zum Herstellen dieses Glases und eine mit diesem Glas versehene Skibrille.

## Beschreibung

Die Erfindung bezieht sich auf ein optisches Glas für Sport-Brillen, auf ein Verfahren zum Herstellen eines solchen Glases sowie auf eine mit wenigstens einem derartige Glas versehene Sport-Brille.

Ski-Brillen sind in den unterschiedlichsten Ausführungen bekannt.

Handelsübliche Ski-Brillen sind mit Intensivfiltern (Grau-, Farb-, Polarisations- und/oder UV-Filtern) ausgestattet und haben gemeinsam, daß sie zwar die Augen schonen, aber aufgrund des stark streuenden und depolarisierenden Mediums Schnee keinerlei Kontrasterhöhungen, insbesondere auch von leichten Unebenheiten der verschneiten Oberfläche, wie beispielsweise Buckel, Dellen, Löcher und Bodenwellen bewirken können.

Der Erfindung liegt die Aufgabe zugrunde, ein optisches Glas für Sport-Brillen sowie eine mit wenigstens einem optischen Glas ausgestattete Sportbrille aufzuzeigen, welches bzw. welche auch bei ungünstigen Lichtverhältnissen eine verbesserte Sicht ermöglicht. Weiterhin liegt der Erfindung die Aufgabe zugrunde, ein Verfahren zum Herstellen eines solchen Glases aufzuzeigen, mit welchem (Verfahren) bei geringem fertigungstechnischen Aufwand verbesserte optische und/oder mechanische Eigenschaften für das Glas erreicht werden.

Zur Lösung dieser Aufgabe sind ein Glas entsprechend dem kennzeichnenden Teil des Patentanspruches 1, ein Verfahren entsprechend dem kennzeichnenden Teil des Patentanspruches 9 und eine Sport-Brille entsprechend dem kennzeichnenden Teil des Patentanspruches 12 ausgeführt.

Bei einer Ausführungsform der Erfindung wird eine Kontrasterhöhung durch eine mehrschichtige oder mehrlagige Ausbildung des Glases erreicht. Hierfür wird das Glas nach einem speziellen Verfahren hergestellt, welches es gestattet, die Materialien für die einzelnen Schichten und/oder die Profilierung dieser Schichten an der gesamten Fläche oder aber nur im Teilbereich so auszubilden, daß sich die speziellen, angestrebten, optischen und/oder mechanischen Eigenschaften für die Schichten und/oder für die Übergänge zwischen zwei angrenzenden Schichten ergeben. Die mehrlagige Struktur des Glases für eine Sport-Brille wird hierbei beispielsweise dadurch erhalten, daß an eine in einem vorausgegangenen Arbeitsgang erzeugte Schicht in einem zeitlich folgenden Arbeitsgang eine angrenzende Schicht angespritzt wird, und zwar derart, daß beide Schichten flächig miteinander verbunden sind. Hierdurch ergibt sich eine wesentlich Vereinfachung der Herstellung. Weiterhin lassen sich hierdurch auch strukturierte Übergänge optisch einwandfrei herstellen.

Bei einer weiteren, bevorzugten Ausführungsform der Erfindung ist das optische Glas so ausgebildet, daß es eine Richtungsfilterung ermöglicht. Auch in diesem Fall ist das Glas laminatartig bzw. mehrlagig ausgebildet und besteht aus einer Vielzahl von Schichten, wobei jede einzelne Schicht sich von der Außenseite des Glases an die Innenseite dieses Glases erstreckt und ein Lichtübertragungselement bildet. Durch die Schichten wird eine Richtungsfilterung der Lichtstrahlung derart erreicht, daß nur Licht, welches in einem bestimmten Winkel auf das Glas auftritt, durch dieses hindurch auf das Auge des Benutzers der Sport-Brille gelangen kann. Die Gläser werden bevorzugt aus einem eine Vielzahl von Schichten aufweisenden Laminat durch Schneiden hergestellt. Letzteres kann, sofern das Material für die lichtdurchlässigen bzw. transparenten oder semi-transparenten Schichten Kunststoff verwendet ist, unter Verwendung des vorgenannten Verfahrens hergestellt werden.

Weiterbildungen der Erfindung sind Gegenstand der Unteran sprüche.

Die Erfindung wird im Folgenden anhand der Figuren an Ausführungsbeispielen näher erläutert. Es zeigen:
- Fig. 1: in vereinfachter und stark vergrößerter Darstellung einen Teilschnitt durch ein Glas einer Ski-Brille gemäß einer ersten, möglichen Ausführungsform;
- Fig. 2: in vereinfachter und stark vergrößerter Darstellung einen Teilschnitt durch ein Glas einer Ski-Brille gemäß einer zweiten, bevorzugten Ausführungsform der Erfindung;
- Fig. 3: eine Draufsicht auf einen Teilbereich des Glases gemäß Fig. 2;
- Fig. 4: in perspektivischer Darstellung ein Ausgangslaminat bzw. eine Flächenschichtstruktur zur Herstellung von Gläsern gemäß Figuren 2 und 3;
- Fig. 5: das Laminat nach Fig. 4, jedoch bei angedeuteten Schrägschnitten für die Herstellung einer Vielzahl von Gläsern aus diesem Laminat;
- Figuren 6 und 7: in perspektivischer Darstellung jeweils einen kleinen Teilbereich eines Glases einer Ski-Sonnenbrille gemäß weiteren Ausführungsformen der Erfindung;
- Fig. 8: in vereinfachter perspektivischer Darstellung eine Kreuzlaminatstruktur zur Herstellung von Gläsern ähnlich der Figuren 6 und 7, zusammen mit angedeuteten Schnittrichtungen.

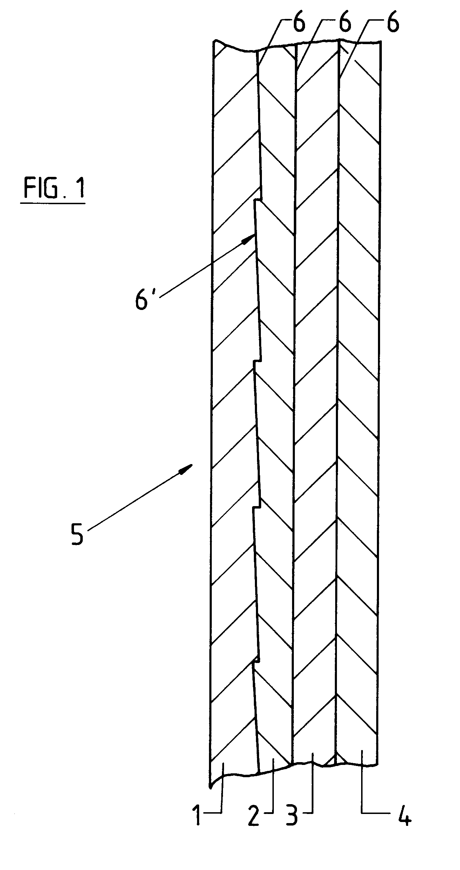

In der Fig. 1 sind 1 - 4 die Schichten eines mehrlagigen optischen Glases 5, welches wenigstens ein Sichtfenster einer ansonsten nicht dargestellten Ski-Brille bildet. Das Glas ist aus Kunststoff in einem Spritzgußwerkzeug hergestellt, und zwar derart, daß die einzelnen Schichten 1 - 4, die jeweils aus einem lichtdurchlässigen bzw. transparenten Kunststoffmaterial bestehen, in einer gemeinsamen Spritzform erzeugt sind. Hierfür wird beispielsweise in einem ersten Verfahrensschritt die Schicht 1 erzeugt, wobei dasjenige Volumen des Innenraums der Spritzform, in welches in zeitlich folgenden Verfahrensschritten das die Schicht 2 - 4 bildende Material eingebracht wird, bei diesem ersten Verfahrensschritt durch drei den Schichten 2 - 4 entsprechende Schieber eingenommen ist. In einem zweiten Verfahrensschritt erfolgt dann das Einbringen des die Schicht 2 bildenden Materials, wobei nur noch das den Schichten 3 und 4 entsprechende Volumen des Innenraumes der Spritzform durch jeweils einen Schieber eingenommen ist. In einem dritten und einem vierten Verfahrensschritt werden dann in entsprechender Weise die Schichten 3 und 4 erzeugt.

Durch das beschriebene Verfahren sowie durch die Auswahl eines geeigneten Kunststoffmaterials sind die Schichten 1 - 4 flächig miteinander verbunden, und zwar ohne daß ein Zwischenmedium erforderlich ist.

Für die Schichten 1 - 4 werden zumindest teilweise unterschiedliche Materialien verwendet, so daß diese Schichten und/oder deren Übergänge 6 unterschiedliche optische und/oder mechanische Charakteristiken aufweisen.

Unter optischen Charakteristiken sind hierbei insbesondere unterschiedliche Filtercharakteristiken durch unterschiedliche Farbgebungen, Wirkung einer oder mehrerer Schichten als Polarisationsfilter usw. zu verstehen.

Weiterhin weist wenigstens eine Schicht, d.h. bei der dargestellten Ausführungsform die beiden Schichten 1 und 2 an ihren Übergangsbereich 6 einen speziellen Schliff bzw. eine spezielle Flächenstrukturierung auf, wie dies in der Figur mit der Linienführung 6' angedeutet ist. Diese Flächenstruktur wird beispielsweise durch eine entsprechende Profilierung desjenigen Schiebers bzw. Einsatzes erzeugt, der bei dem vorgenannten ersten Verfahrensschritt den der später zu erzeugenden Schicht 2 entsprechenden Teil des Innenraums der Spritzform einnimmt.

Die optischen Eigenschaften der einzelnen Schichten 1 - 4 sind beispielsweise so ausgewählt, daß die das mehrlagige Glas 5 (Scheibe) aufweisende Ski-Brille eine besonders gute Sicht auch bei schlechten Sichtverhältnissen ermöglicht.

Die mechanischen Eigenschaften der einzelnen Schichten 1 - 4 sind beispielsweise so ausgewählt, daß die Ski-Brille zumindest an der Außenseite des mehrlagigen bzw. mehrschichtigen Glass beispielsweise gegen mechanische Krafteinwirkung (wie Stöße, Schläge usw.) weitestgehend unempfindlich ist und hohe Druckbelastungen aufnehmen kann.

Die Erfindung wurde voranstehend an einem ersten Ausführungsbeispiel beschrieben, wobei Änderungen sowie Abwandlungen möglich sind. So ist es beispielsweise möglich, daß das mehrlagige Glas mehr oder weniger als vier Schichten aufweist. Weiterhin ist es möglich, die Schichten 1 - 4 in einer einzigen Form dadurch herzustellen, daß nach dem Fertigstellen einer Schicht der Formraum für eine angrenzende Schicht nicht durch Entfernen eines Einsatzes oder eines Schiebers, sondern dadurch gebildet wird, daß die verwendete Spritzform eine bewegliche bzw. verschiebbare Wandung bzw. Formfläche aufweist, die beim Verschieben den Formraum für die jeweils anzuschließende Schicht frei gibt.

Weiterhin ist es auch möglich, in einem Verfahrensschritt zwei Schichten, beispielsweise die beiden Schichten 1 und 3 zu erzeugen, und dann in einem darauffolgenden Verfahrensschritt zumindest eine zwischen diesen beiden Schichten liegende dritte Schicht, beispielsweise die Schicht 2 herzustellen.

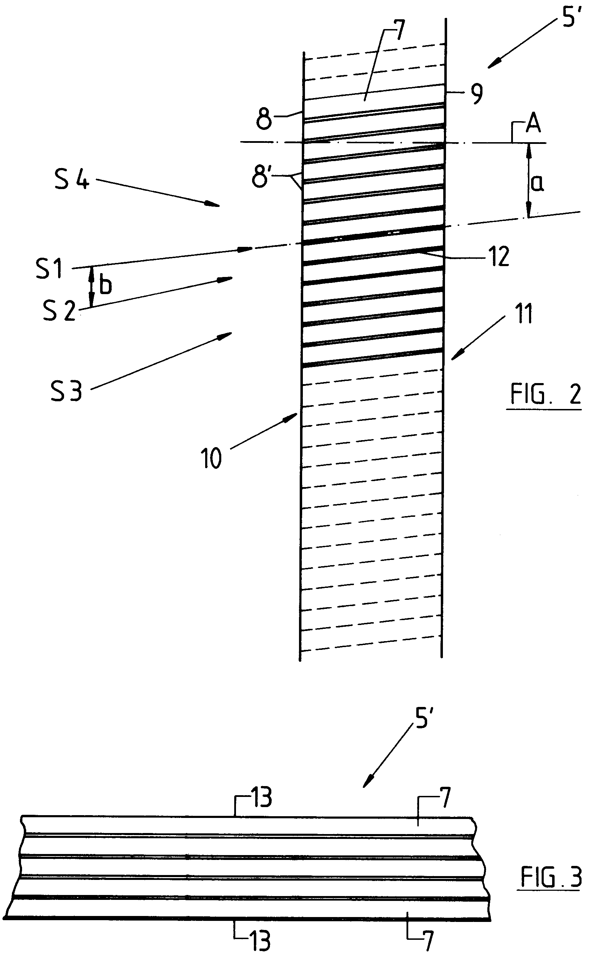

Die Figuren 2 und 3 zeigen ein Glas 5', welches für eine Ski-Brille bestimmt ist und eine Verbesserung der Sicht bzw. des Kontrastes auch bei sehr schrägem Lichteinfall bringt.

Das Glas 5' besteht aus einer Vielzahl von Schichten 7 aus einem lichtdurchlässigen bzw. transparenten oder semi-transparenten Material, beispielsweise aus Glas oder einem geeigneten Kunststoff. Die Schichten 7 besitzen in dem in der Fig. 2 dargestellten Schnitt durch das Glas 5' eine etwa parallelogrammartige Querschnittsform in der Weise, daß die Schichten 7 mit den kürzeren Querschnittsseiten 8 und 9 auf der Außenfläche 10 bzw. Innenfläche 11 des Glases 5' liegen und die einzelnen Schichten 7 an den längeren Querschnittsseiten 12 aneinander anschließen und miteinander verbunden sind, beispielsweise durch einen geeigneten lichtdurchlässigen Kleber oder bei aus Kunststoff bestehenden Schichten 7 beispielsweise dadurch, daß diese Schichten, die in einem Spritz- oder Formverfahren zeitlich aufeinanderfolgend hergestellt sind, durch das diese Schichten bildende Material chemisch und/oder mechanisch miteinander verbunden sind. Bevorzugt sind die Schichten 7 zumindest an den größeren Querschnittsseiten 12, d.h. dort, wo die Schichten aneinander anschließen, mit einer Anti-Reflexbeschichtung versehen.

Die Schichten 7 erstrecken sich, wie in der Fig. 3 angedeutet ist, über die gesamte senkrecht zur Zeichenebene der Fig. 2 verlaufende Breite des Glases 5'. Die größeren Querschnittsseiten 12 bzw. die entsprechenden oberen und unteren Flächen der Schichten 7 schließen mit der optischen Achse A, die bei dem Glas 5' senkrecht zur Außenfläche 10 bzw. Innenfläche 11 verläuft, einen Winkel a ein, der etwa 2 - 12^{o} beträgt. In der Ski-Brille wird das Glas 5' derart montiert, daß die Schnittkanten 13 horizontal oder in etwa horizontal angeordnet sind und jede Querschnittsseite 12 ausgehend von der Innenfläche 11 zur Außenfläche 10 schräg nach unten verläuft, und zwar im Winkel a.

Mit dem Glas 5' wird eine Richtungsfilterung der ins Auge gelangenden Lichtstrahlung erreicht, die von einer Lichtquelle (in der Regel Sonne) ausgehend an dem unebenen, verschneiten Gelände reflektiert, gestreut oder gebeugt wurde. Bei sehr schräg einfallender Lichtstrahlung wirken alle auch noch so rauhen Oberflächen reflektierend. Weiterhin wird bei sehr schräg einfallender Lichtstrahlung auch die Projektions-Schattenwirkung in ihrer Dimension vergrößert.

Durch den beschriebenen laminaren Aufbau des Glases 5' wird nun erreicht, daß durch dieses Glas und damit an das Auge nur solches Licht gelangen kann, dessen Strahlungsrichtung innerhalb eines bestimmten Winkelbereichs liegt, wie dies mit den Richtungspfeilen S1 und S2 bzw. mit dem zwischen diesen Pfeilen gebildeten Winkel b angedeutet ist, während Lichtstrahlung aus anderen Richtungen das Glas 5' nicht passieren kann und vom Auge ferngehalten wird, wie dies mit den Richtungspfeilen S3 und S4 angedeutet ist. Durch die Anti-Reflexbeschichtungen an den größeren Querschnittsseiten 12 bzw. an den entsprechenden Flächen der Schichten 7 werden dort Mehrfach-Reflexionen und hierdurch bedingte Reflexbilder geschwächt oder vollständig vermieden.

Durch diese Richtungsfilterung wird erreicht, daß nur dasjenige Licht ins Auge gelangen kann, das Projektionsschatten erzeugt, während das übrige, reflektierte oder gestreute Licht weitestgehend ausgefiltert ist, so daß der Kontrast der Projektionsschatten gegenüber der stark in alle Richtung streuenden Umgebung erhöht wird.

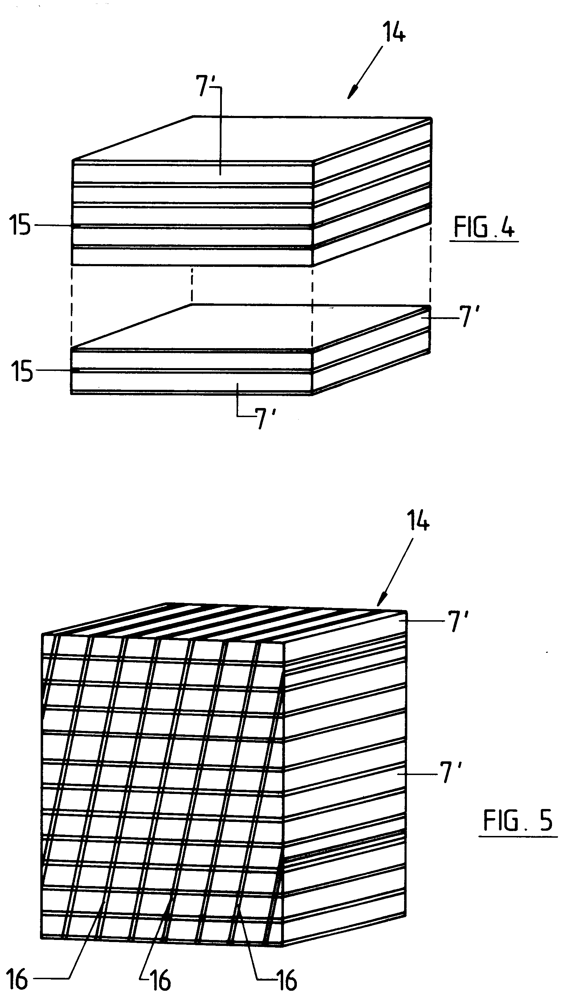

Das Glas 5' läßt sich in besonders einfacher Weise aus dem in der Fig. 4 dargestellten Ausgangslaminat 14 herstellen, welches aus mehreren stapelartig übereinander angeordneten und flächig miteinander verbundenen Schichten 7' aus dem transparenten bzw. semi-transparenten Material besteht. An den Übergängen 15 zwischen den Schichten 7' sind die Anti-Reflexbeschichtungen vorgesehen. Die Schichten 7' sind in der Weise, wie dies vorstehend für die Schichten 7 des Glases 5' beschrieben wurde, miteinander verbunden.

Zum Herstellen der Gläser 5' wird das quaderförmige Laminat 14 schräg geschnitten, und zwar derart, daß die entsprechenden, jeweils parallelen Schnitte 16 in Ebenen liegen, die mit den Übergängen 15 den Winkel 90^{o} minus a einschließen, und die Schnittlinie zwischen der Ebene jedes Schnittes 16 und den Übergängen der Schichten 7' senkrecht zu zwei Seiten des rechteckförmigen Zuschnitts der Schichten 7' verläuft.

Grundsätzlich ist es möglich, die durch Schneiden des Laminats 14 gewonnenen Gläser 5' mit einer herkömmlichen Filter- bzw. Augenschutz-Beschichtung versehen werden. Grundsätzlich ist es aber auch möglich, daß das die Schichten 7 bzw. 7' bildende Material bereits als Filter (UV-Filter) ausgebildeet ist und/oder entsprechende Schutzfaktoren enthält und/oder gefärbt und/oder als Polarisationsfilter ausgeführt ist.

Unabhängig hiervon ist es weiterhin auch möglich, die aus dem Laminat 14 durch Schneiden gewonnenen Gläser 5' entsprechend der jeweils erforderlichen Brillen-Dioptrienzahl zu schleifen und anschließend zu polieren.

Zur Vermeidung von Moiree-Effekten ist es weitehin auch möglich, das Glas 5' bzw. die dortigen Schichten 7 so auszubilden, daß diese an den an der Außenseite 10 vorgesehenen Querschnittsseiten 8 konvex gewölbt sind, wie dies in der Fig. 2 bei 8' mit unterbrochenen Linien angedeutet ist. Diese Linsenform (Zylinderlinsen-Form) kann z.B. durch Preßvorgänge oder unter Verwendung von lithographischen Verfahren hergestellt werden.

Der Winkel a, der, wie oben angegeben, im Bereich zwischen etwa 2 - 12^{o} liegt, ist bevorzugt unterschiedlich für Kinder und Erwachsene so eingestellt, daß eine Voraus-Sicht von ca. 10 - 20 m mit der mit dem Glas 5' ausgestatteten Ski-Brille möglich ist.

Ein Vorteil des Glases 5' besteht darin, daß trotz der Erhöhung des Kontrastes und damit trotz verbesserter Sicht insbesondere bei sehr schräg einfallender Lichtstrahlung durch die sich über die gesamte Breite des Glases 5' erstreckenden Schichten 7 in Seitenrichtung ein großer Blickwinkel erhalten bleibt und die Richtungsfilterung im wesentlich eine Oben/Unten-Filterung ist.

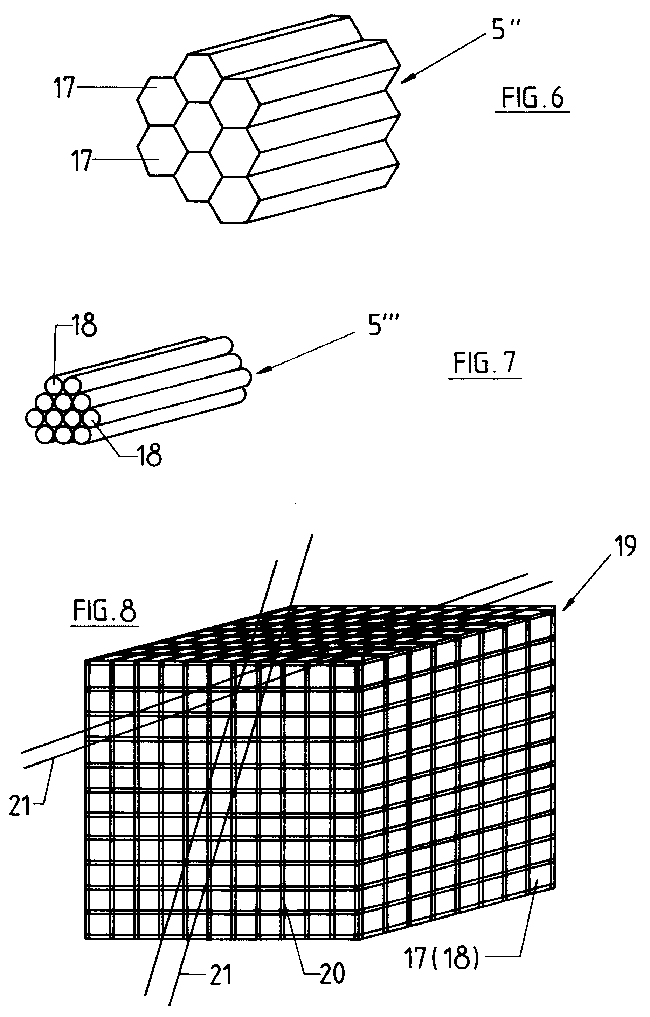

Die Figuren 6 und 7 zeigen als weitere Ausführungsbeispiele Gläser 5'' und 5''', die sowohl eine Oben-Unten-Richtungsfilterung als auch eine seitliche Richtungsfilterung bewirken. Das Glas 5'' setzt sich aus einer Vielzahl von prismenförmigen, lichtübertragenden Elementen zusammen, die bei dem Glas 5'' eine sechseckförmige Querschnittsform aufweisen, bienenwabenartig dicht aneinander anschließend zu dem Glas miteinander verbunden sind und mit ihren Enden jeweils die Innenfläche bzw. Außenfläche des Glases bilden und mit ihrer Längserstreckung jeweils parallel zur optischen Achse bzw. senkrecht zur Innenfläche bzw. Außenfläche des Glases 5'' liegen. Bei dem Glas 5''' sind anstelle der Elemente 17 Elemente 18 vorgesehen, die kreiszylinderförmig ausgebildet sind. Alle Flächen der Gläser 5'' und 5''', insbesondere auch die Grenzflächen zwischen den Elementen 17 bzw. 18 sind wiederum mit der Anti-Reflexbeschichtung versehen. Zum Herstellen der Gläser 5'' bzw. 5''' dient ein Kreuzlaminat 19, welches im einfachsten Fall aus einer Vielzahl von an ihren Längsseiten miteinander verbundenen Profillängen eines den Elementen 17 und 18 entsprechenden Profils besteht. Aus dem Kreuzlaminat 19 werden dann die Gläser 5'' bzw. 5''' herausgeschnitten und zwar mit Schnitten, wie sie durch die Schnittlinien 20 und 21 angedeutet sind. Verlaufen die Schnitte des Kreuzlaminats 19 schräg, so schließen dann auch die Achsen der Elemente 17 bzw. 18 mit der optischen Achse des betreffenden Glases einen entsprechenden Winkel ein.

Die Dicke der Schichten 7 bzw. die Querschnittsabmessungen der Elementen 17 und 18 liegen beispielsweise in der Größenordnung von 0,5 mm.

Die optische Achse A ist bei den Gläsern 5, 5', 5'', 5''' bei planer Ausbildung die senkrecht zu den Oberflächenseiten dieses Glases verlaufende Achse. Sind die Gläser für eine bestimmte Brillen-Dioptrienzahl geschliffen, so ist die optische Achse A die Achse der durch diesen Schliff erzielten Linse oder die Mittelachse des Glases einer Ski-Brille.

## Patentansprüche

1. Optisches Glas für eine Sport-Brille, insbesondere Ski-Brille, **dadurch gekennzeichnet,** daß das Glas (5, 5', 5'', 5''') aus einer Vielzahl von laminatartig miteinander verbundenen Licht übertragenden Elementen (7, 17, 18) aus transparentem oder semi-transparentem Material besteht, und zwar derart, daß das Glas in wenigstens einer eine optische Achse des Glases einschließenden Ebene eine Richtungsfilterung für die auf das Glas auftreffende Lichtstrahlung bewirkt.

2. Glas nach Anspruch 1, dadurch gekennzeichnet,
daß die Elemente (7, 17, 18) an ihren Flächen, insbesondere an ihren jeweils den Übergang zu einem benachbarten Element (7, 17, 18) bildenden Flächen mit einer Anti-Reflexbeschichtung versehen sind,
und/oder daß auf das Glas eine Augenschutz-Beschichtung aufgebracht ist, vorzugsweise zur Bildung eines Grau-, Farb-, Polarisations- und/oder UV-Filters,
und/oder daß das die Elemente (7, 17, 18) bildende Material als Grau-, Farb-, Polarisations- und/oder UV-Filter ausgebildet ist,
und/oder daß es entsprechend einer erforderlichen Brillen-Dioptrienzahl geschliffen ist.

3. Glas nach Anspruch 1 oder 2, dadurch gekennzeichnet, daß die Elemente (1 - 4, 7) von einer Vielzahl von Schichten (1 - 4, 7) gebildet sind, die laminatartig flächig miteinander verbunden sind und aneinander anschließen.

4. Glas nach Anspruch 3, dadurch gekennzeichnet, daß die Übergänge zwischen den Schichten (7) parallel zur optischen Achse liegen oder mit dieser einen Winkel kleiner als 90^{o} einschließen, wobei beispielsweise die Übergänge (12) mit der optischen Achse (A) einen Winkel (a) kleiner als 20^{o}, vorzugsweise einen Winkel von etwa 2 - 12^{o} einschließen.

5. Glas nach einem der Ansprüche 1 - 4, dadurch gekennzeichnet, daß jede Schicht (7) mit einer Querschnittsseite (8) bzw. mit der entsprechenden Seitenfläche einen Teil einer Außenfläche (10) des Glases (5') und mit einer weiteren Querschnittsseite bzw. mit der betreffenden Seitenfläche einen Teil der Innenfläche (11) des Glases bilden.

6. Glas nach einem der Ansprüche 1 - 5, dadurch gekennzeichnet, daß die Dicke der Schichten (7) kleiner ist als die Dicke des Glases (5').

7. Glas nach einem der Ansprüche 1 - 6, dadurch gekennzeichnet, daß die Schichten (7) aus Mineralglas, aus Kunststoff oder aus Kunststoff-Folien bestehen, und/oder daß für angrenzende Schichten (1 - 4) unterschiedliche Kunststoffmaterialien verwendet sind, und/oder daß wenigstens eine Schicht (1, 2) an wenigstens einer Seite und/oder wenigstens ein Übergang (6) zwischen zwei angrenzenden Schichten (1, 2) strukturiert ist.

8. Glas nach einem der Ansprüche 1 - 7, dadurch gekennzeichnet, daß die Elemente stäbchenförmige Elemente (17, 18) sind, die an ihren Umfangsflächen aneinander anschließen und miteinander verbunden sind,
wobei vorzugsweise die Elemente (17, 18) einen von einem Vieleck, bevorzugt von einem Sechseck gebildeten Querschnitt oder einen kreisförmigen oder ovalen Querschnitt aufweisen und/oder mit ihren Achsen parallel zueinander angeordnet sind,
und/oder wobei vorzugsweise die Elemente (17, 18) mit der optischen Achse (A) einen Winkel kleiner als 45^{o}, vorzugsweise kleiner als 20^{o}, bevorzugt einen Winkel zwischen etwa 2 -12^{o} einschließen,
und/oder wobei vorzugsweise die Elemente (7, 17, 18) an der Außenfläche (10) des Glases (5', 5'', 5''') konvex gewölbt sind.

9. Verfahren zum Herstellen eines optischen Glases für eine Sport-Brille, insbesonder Ski-Brille, dadurch gekennzeichnet, daß das Glas oder ein Ausgangslaminat (14, 19) zum Herstellen des Glases mit einer Vielzahl von flächig miteinander verbundenen Schichten (1 - 4, 7; 7') oder mit einer Vielzahl von an ihren Umfangsflächen aneinander anschließenden und miteinander verbundenen stabförmigen Elementen (17, 18) aus einem transparenten oder semi-transparenten Material hergestellt wird.

10. Verfahren nach Anspruch 9, dadurch gekennzeichnet, daß vor dem Herstellen des Ausgangslaminats oder beim Bilden des Laminats (14, 19) die Schichten und/oder stabförmigen Elemente (17, 18) zumindest an ihren anschließenden Flächen mit einer Anti-Reflexbeschichtung versehen werden,
und/oder daß das Ausgangslaminat (14, 19) durch Schnitte (16, 20, 21) senkrecht oder quer zur Laminierung, vorzugsweise durch Schnitte, die mit den Ebenen der Anschlußflächen zwischen den Schichten (7') oder mit der Längserstreckung der stabförmigen Elemente (17, 18) einen Winkel etwa im Bereich zwischen 70 und 90^{o}, vorzugsweise einen Winkel im Bereich zwischen etwa 78 und 88^{o} einschließen,
und/oder daß das optische Glas oder das Ausgangslaminat (14, 19) durch ein Spritzverfahren dadurch hergestellt werden, daß in einem ersten Verfahrensschritt in einer Spritzform jeweils eine Schicht erzeugt und in einem zeitlich folgenden Verfahrensschritt eine an diese eine Schicht anschließende weitere Schicht angespritzt wird,
wobei das Formen des mehrschichtigen Glases (5) oder des Laminats (14) in einer einzigen Spritzform in zeitlich aufeinanderfolgenden Verfahrensschritten erfolgt.

11. Verfahren nach Anspruch 9 oder 10, gekennzeichnet durch Verwendung von unterschiedlichen, transparenten Kunststoffmaterialien zur Erzeugung der Schichten (1 - 4) des Glases (5) oder des Laminats (14, 19) und/oder zur Erzeugung von Übergängen (6) mit optisch und/oder mechanisch unterschiedlichen Charakteristiken,
wobei vorzugsweise für angrenzende Schichten unterschiedliche Kunststoffmaterialien verwendet werden,
und/oder wobei vorzugsweise wenigstens eine Schicht (1, 2) an wenigstens einer Seite und/oder wenigstens ein Übergang (6) zwischen zwei angrenzenden Schichten (1, 2) strukturiert ist.

12. Sport-Brille, insbesondere Ski-Brille, hergestellt unter Verwendung wenigstens eines optischen Glases nach einem der Ansprüche 1 - 18, wobei das optische Glas (5, 5', 5'', 5''') so orientiert ist, daß zumindest eine Richtungsfilterung der auftreffenden Lichtstrahlung in einer die optische Achse einschließenden vertikalen Ebene vorliegt.
